# EUROPEAN PATENT SPECIFICATION

(11) **EP 0 314 147 B1**
(45) Date of publication and mention of the grant of the patent: **22.12.1993**
(21) Application number: 88117950.1
(22) Date of filing: 27.10.1988
(51) Int. Cl.: C07C 239/10, C07D 295/28

(54) **Process for the synthesis of N,N-dialkyl-Hydroxylamines**
Verfahren zur Synthese von N,N-Dialkylhydroxylaminen
Procédé de synthèse de N,N dialcoyl hydroxylamines

(30) Priority: 29.10.1987 IT 2245287
(43) Date of publication of application: 03.05.1989
(73) Proprietor: ENICHEM S.p.A., 20124 Milano (IT)
(72) Inventor: Tonti, Sergio, Dr., I-30173 Mestre (VE) (IT); Roffia, Paolo, Dr., I-21047 Saronno (VA) (IT); Cesana, Alberto, Dr., I-20048 Carate Brianza (MI) (IT); Mantegazza, Maria Angela, Dr., I-20040 Cambiago (MI) (IT); Padovan, Mario, I-20100 Milano (IT)
(74) Representative: Weinhold, Peter, Dr.

(56) References cited:
- EP-A- 0 233 622
- EP-A- 0 267 362
- BE-A- 615 736
- FR-A- 1 436 118

## Description

Several methods for synthesizing N,N-dialkyl-hydroxylamines are already known. Particularly, the nitrones can be reduced either to disubstituted hydroxylamines by means of lithium aluminum hydride or by means of potassium boro-hydride or by hydrogenation on platinum black (Coll. Czech. Chem. Comm. 2C, 202 (1955), JACS 79, 5739 (1957); 78, 6208 (1956); Gazz. Chim. Ital. 51, II, 306 (1921)). The pyrolysis of trialkylamine oxides, known as Cope reaction, is useful for the synthesis of N,N-dialkyl-hydroxylamines as well.

Should the amine oxide have more than one alkyl group capable of forming an olefin, a mixture of hydroxylamines is obtained (Org. Synthesis Coll. Vol. IV; 612 (1963)).

The N,N-dialkyl-hydroxylamines may also be prepared either by reaction of compounds containing a N-O bond, by allowing said compounds to react with organometallic compounds (J.Chem. Soc. 119, 251 (1921)) or by alkylation of hydroxylamines or N-alkylhydroxylamines with alkyl halogenides (J. Org. Chem. 28, 1068 (1963); US-A-3491151).
It is also known that secondary amines, when treated with hydrogen peroxide or with acylperoxides, give rise to the formation of N,N-dialkyl-hydroxylamines (Chem. Ber. 65, 1799 (1932); Arch. Pharm. 299, 166 (1966); JACS 72, 2280 (1950); J. Chem. Soc. 3144 (1963)). The reaction is of a general type and can be used with primary amines as well. The modalities involved up till now proved to be, however, extremely unsatisfactory, owing the low yield in the desired product. Moreover, the oxidation of the carbon atoms in alpha position, with respect to nitrogen, gave rise to the formation of a complex mixture of products. The oxidation of secondary amines with hydrogen peroxide was carried out in the presence of a reaction promoter as well, in particular in the presence of an ester of formic acid (DE-A-1004191) or in the presence of a usual catalyst, containing Mo, W and the like (BE-A-615,736). In each case the yields are low, whereas the decomposition of hydrogen peroxide clearly prevails over the formation of hydroxylamine. Also the above mentioned preparation processes for N,N-dialkylamines which are not based on oxidation by means of hydrogen peroxide, are characterized by the need for expensive reactants, by the involvement of not very stable compounds, by the formation of a large number of by-products and difficulties in isolating the desired product.

It has now surprisingly been found that the preparation process for N,N-dialkylhydroxylamines (in particular of N,N-diethyl-hydroxylamine) can be very much improved by carrying out the oxidation of the corresponding amine by means of hydrogen peroxide in the presence of a particular catalyst.

In its broadest sense the invention resides in a process for the synthesis of N,N-dialkyl-hydroxylamines of general formula (I): 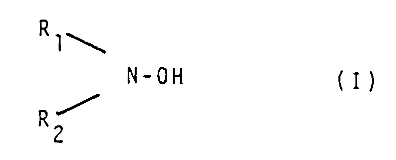
wherein R₁ and R₂, which may be the same or different, represent an alkyl, cycloalkyl, alkyl-cycloalkyl or cycloalkylalkyl group having from 1 to 40 carbon atoms or are part of a cycloaliphatic ring containing the hetero-atom N and having from 4 to 8 carbon atoms, by reaction with hydrogen peroxide of the corresponding (secondary) dialkyl-amines of general formula (II): 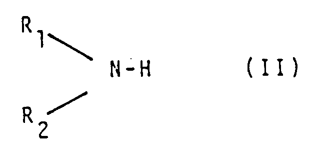
which is characterized in that said reaction is carried out in the presence of a catalyst based on titanium-silicalite.

The term "titanium-silicalite" is described and defined in e.g. EP-A-299430 and in EP-A-267362 and in the documents mentioned therein. The disclosure of said applications, as far as it relates to titanium-silicalites, is incorporated herein.

Extraordinary results may be obtained starting from secondary amines in which R₁ and R₂ each contain from 1 to 8 carbon atoms, or from heterocyclic compounds of pyrrolidinic or piperidinic nature, particularly diethyl-amine, dipropyl-amine, dioctylamine; N-methyl-, N-ethyl-amine and pyrrolidine.

Further examples of preferred groups R₁ and R₂ are: butyl, pentyl, hexyl, heptyl (alkyl groups may be straight-chain or branched); cyclopentyl, cyclohexyl, cyclooctyl; methylcyclopentyl, dimethylcyclopentyl, methylcyclohexyl, dimethylcyclohexyl; cyclopentylmethyl and cyclohexylmethyl. When R₁ and R₂ combine to form a heterocyclic ring they may represent e.g. butylene, pentylene, hexylene and 3-oxa-pentylene.

The practical interest in hydroxylamines is due to their use in different fields as reducing agents, stabilizers or polymerization inhibitors.

In particular, on account of these specific properties, these compounds may satisfactorily replace hydrazine derivatives as deoxygenating agents for water which is to be fed into thermal plants or in general into steam generating plants. By the process according to the invention a high yield with respect to the hydrogen peroxide (generally between 85 and 95%), a high selectivity with respect to the starting amine (higher than 90 and sometimes even higher than 95%) and a practically quantitative conversion of the starting amine can be obtained.

The hydroxylation process for the secondary amines by means of H₂O₂ may be carried out in different ways; for instance, it may be operated either in the absence or in the presence of a solvent, said solvent being e.g. water or a suitable organic solvent miscible with water, such as the aliphatic alcohols, or mixtures thereof. Particularly good results have been obtained by using as solvent a tertiary alcohol which is practically inert with respect to the oxidizing system; really extraordinary results have been obtained with t-butyl or t-amyl alcohol. The temperature generally ranges from 25 to 150°C, preferably from 40 to 120°C. The hydroxylation of the dialkylamines may generally be carried out at atmospheric pressure or (preferably) at superatmospheric pressure, in order to keep both solvent and reactants in the liquid phase. The catalyst is preferably used in a form finely dispersed in the reaction medium, in amounts ranging from 0.1 to 50 parts by weight (preferably from 1 to 30 parts) per 100 parts of dialkylamine. The weight ratio of dialkylamine to solvent generally ranges from 1 to 30 parts, preferably from 1 to 20 parts by weight of amine per 100 parts of solvent. The reaction stoichiometry requires an amount of hydrogen peroxide equivalent to the amount of amine; generally the molar ratio of the two reactants (hydrogen peroxide: dialkylamine) ranges from 0.9 to 1.2, preferably from 0.9 to 1.1.

The process according to the invention may be carried out either in a semicontinuous way (by feeding only hydrogen peroxide continuously to the reactor) or in a continuous way (by continuously feeding both the reactants to the reactor). The reactor effluent is a suspension that needs to be filtered in order to recover the catalyst (which may be recycled to the reaction); if the filtration is carried out inside the reactor the recovered effluent consists of a solution of the starting amine, the reaction product, the reaction water and the solvent. The different components can be isolated from this solution by known methods (distillation, crystallization, extraction and the like). Non-converted reactants and solvent may be recycled to the hydroxylation reaction, whereas the reaction product is recovered and optionally subjected to other purification operations, according to the desired degree of purity.

The following examples illustrate the invention, without limiting, however the scope thereof.

### Example 1

A glass reactor equipped with a stirrer and heating jacket was pressurized with nitrogen, after a vacuum had been applied by means of a mechanical pump; said reactor was charged with 1.5 g of a finely subdivided powder, obtained by grinding a titanium silicalite (prepared according to example 2 of EP-A-267362) with 7.21 g of diethylamine in 50 ml of t-butyl alcohol. The temperature was gradually increased by feeding a thermostatic liquid of a temperature of 80°C to the reactor jacket. At this point hydrogen peroxide (as a 30% by weight aqueous solution/was added. The addition was carried out over 35 minutes, adding a total of 5.97 g of dilute H₂O₂, corresponding to 0.056 moles of pure H₂O₂. Thereafter the solution was cooled and directly analysed. The non-converted diethylamine accounted for 3.49 g, whereas the formed N,N-diethylhydroxylamine amounted to 4.32 g, which corresponds to a 51.5% conversion, with a 95.5% selectivity to N,N-diethylhydroxylamine; the hydrogen peroxide conversion was practically complete, with a yield to N,N-diethylhydroxylamine of 87.1%.

### Example 2

Example 1 was repeated, increasing the hydrogen peroxide amount to 9.63 g, corresponding to 0.090 moles, and by carrying out the addition over 54 minutes. The obtained results were as follows:

| | |
|---|---|
| - diethylamine conversion | 80.4% |
| - amine selectivity to N,N-diethyl-hydroxylamine | 92.3% |
| - N,N-diethyl-hydroxylamine yield (with respect to H₂O₂) | 80.9% |
| - hydrogen peroxide conversion | 99.8% |

### Example 3

Example 2 was repeated, increasing the t-butanol amount to 100 ml and keeping unchanged the other reactants and the reaction conditions. The obtained results were as follows:

| | |
|---|---|
| - diethylamine conversion | 84.5% |
| - amine selectivity to N,N-diethyl-hydroxylamine | 88.7% |
| - N,N-diethyl-hydroxylamine yield (with respect to H₂O₂) | 78.3% |
| - hydrogen peroxide conversion | 99.4% |

### Example 4

Example 1 was repeated, adding 25 ml of H₂O₂ and 25 ml of t-butanol (as the dispersing medium for the catalyst) and changing the reaction temperature (to 60°C); the obtained results were as follows:

| | |
|---|---|
| - diethylamine conversion | 40.9% |
| - amine selectivity to N,N-diethyl-hydroxlamine | 54.2% |
| - N,N-diethyl-hydroxylamine yield (with respect to H₂O₂) | 41.1% |
| - H₂O₂ conversion | 91.6% |

### Example 5 (comparative example)

Example 1 was repeated, omitting the addition of the catalyst. The obtained (very poor) results were as follows:

| | |
|---|---|
| - diethylamine conversion | 23.4% |
| - selectivity to N,N-diethyl-hydroxylamine | 17.3% |
| - N,N-diethyl-hydroxylamine yield (with respect to H₂O₂) | 7.2% |
| - hydrogen peroxide conversion | 66.9% |

### Example 6

The apparatus used in Example 1 was charged with 7.4 g of pyrrolidine, 50 ml of t-butyl alcohol and 1.5 g of the same (finely subdivided) titanium-silicalite. The suspension, kept under stirring by means of a magnetic stirrer, was heated at 80°C. Thereafter the addition of dilute hydrogen peroxide (at 30% by weight) by means of a metering pump was commenced. The addition was completed within 150 minutes; the total amount of added H₂O₂ was 0.054 moles. When the addition was over, the solution was cooled and analysed. The obtained results were as follows:

| | |
|---|---|
| - pyrrolidine conversion | 30.5% |
| - pyrrolidine selectivity to N-hydroxy-pyrrolidine | 30.4% |
| - N-hydroxy-pyrrolidine yield (with respect to H₂O₂) | 18.0% |
| - hydrogen peroxide conversion | 99.7% |

### Example 7 (comparative example)

Example 6 was repeated without using any catalyst; the obtained (very poor) results were as follows:

| | |
|---|---|
| - pyrrolidine conversion | 25.4% |
| - pyrrolidine selectivity to N-hydroxy-pyrrolidine | 0.2% |
| - N-hydroxy-pyrrolidine yield (with respect to H₂O₂) | 0.1% |
| - hydrogen peroxide conversion | 93.6% |

## Claims

1. Process for the synthesis of N,N-dialkyl-hydroxylamines of general formula (I) 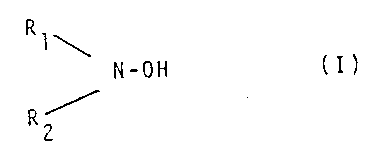 wherein R₁ and R₂, which may be the same or different, represent an alkyl, cycloalkyl, alkyl-cycloalkyl or cycloalkyl-alkyl group having from 1 to 40 carbon atoms or are part of a cycloaliphatic ring containing the heteroatom N and having from 4 to 8 carbon atoms, by reaction with hydrogen peroxide of the corresponding (secondary) dialkyl-amines of general formula (II) 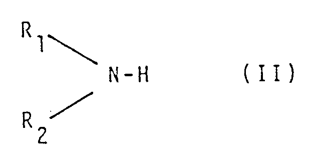 characterized in that said reaction is carried out in the presence of a catalyst based on titanium-silicalite.

2. Process according to claim 1, wherein said secondary amine contains alkyl groups having from 1 to 8 carbon atoms or is a heterocyclic compound of pyrrolidinic or piperidinic nature.

3. Process according to claim 2, wherein said amine is selected from the group of dimethylamine; diethylamine: N-methyl, N-ethyl-amine; dipropyl-amine; dioctylamine and pyrrolidine.

4. Process according to anyone of the preceding claims, wherein said reaction is carried out in a solvent selected from the group of water, aliphatic alcohols and mixtures thereof, in a weight ratio amine: solvent of from 1:100 to 30:100, the reaction temperature ranging from 25 to 150°C.

5. Process according to anyone of the preceding claims, wherein the amount of catalyst ranges from 0.1 to 50 parts by weight per 100 parts of amine.

6. Process according to anyone of the preceding claims, wherein the H₂O₂:amine molar ratio ranges from 0.9 to 1.2.

## Patentansprüche

1. Verfahren zur Synthese von N,N-Dialkylhydroxylaminen der allgemeinen Formel (I) 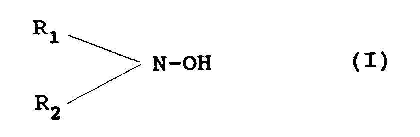 worin R₁ und R₂, die gleich oder verschieden sein können, eine Alkyl-, Cycloalkyl-, Alkyl-cycloalkyl- oder Cycloalkyl-alkylgruppe mit 1 - 40 Kohlenstoffatomen bedeuten oder Teil eines cycloaliphatischen Ringes mit dem Heteroatom N und 4 - 8 Kohlenstoffatomen sind, durch Umsetzung mit Wasserstoffperoxid der entsprechenden (sekundären) Dialkylamine der allgemeinen Formel (II) 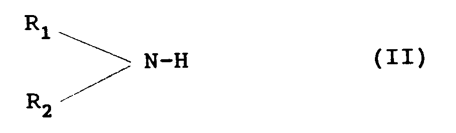 dadurch gekennzeichnet, daß die genannte Reaktion in Anwesenheit eines Katalysators auf der Basis von Titansilicalit erfolgt.

2. Verfahren nach Anspruch 1, worin das genannte sekundäre Amin Alkylgruppen mit 1-8 Kohlenstoffatomen enthält oder eine heterocyclische Verbindung des Pyrrolidin- oder Piperidin-Typs ist.

3. Verfahren nach Anspruch 2, worin das genannte Amin ausgewählt wird aus der Gruppe Dimethylamin; Diethylamin: N-Methyl-, N-Ethylamin; Dipropylamin; Dioctylamin und Pyrrolidin.

4. Verfahren nach einem der vorhergehenden Ansprüche, worin die genannte Reaktion in einem Lösungsmittel erfolgt, ausgewählt aus der Gruppe: Wasser, aliphatische Alkohole und Mischungen von diesen, in einem Gewichtsverhältnis von Amin : Lösungsmittel von 1: 100 bis 30 : 100, und die Reaktionstemperatur von 25 bis 150° C beträgt.

5. Verfahren nach einem er vorhergehenden Ansprüche, worin die Katalysatormenge von 0,1 bis 50 Gew.-Teile pro 100 Teile Amin beträgt.

6. Verfahren nach einem der vorhergehenden Ansprüche, worin das Molverhältnis von H₂O₂ : Amin von 0,9 bis 1,2 beträgt.

## Revendications

1. Procédé de synthèse des N,N-dialkyl-hydroxylamines représentées par la formule générale (I): 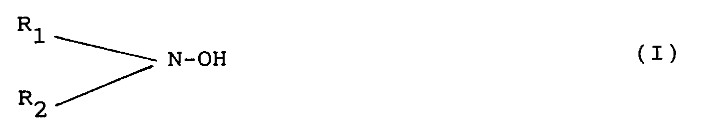 dans laquelle:
R₁ et R₂, qui peuvent être identiques ou différents l'un de l'autre, représentent un radical alkyle, cycloalkyle, alkyl-cycloalkyle ou cycloalkyl-alkyle, comportant de 1 à 40 atomes de carbone, ou ils représentent un cycle cycloaliphatique contenant l'hétéroatome N et comportant de 4 à 8 atomes de carbone,
par réaction des dialkylamines (secondaires) correspondantes de formule générale (II): 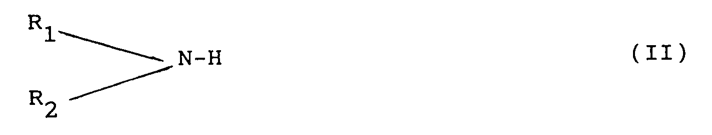 avec du peroxyde d'hydrogène,
caractérisée en ce que ladite réaction est mise en oeuvre en présence d'un catalyseur à base de silicalite-titane.

2. Procédé selon la revendication 1, caractérisé en ce que ladite amine secondaire comporte des radicaux alkyle renfermant de 1 à 8 atomes de carbone, ou correspond à un dérivé hétérocyclique de nature pyrrolidinique ou piperidinique.

3. Procédé selon la revendication 2, caractérisé en ce que ladite amine est sélectionné dans le groupe consistant en diméthylamine, diéthylamine, N-méthyle, N-éthylamine; dipropylamine; dioctylamine et pyrrolidine.

4. Procédé selon l'une quelconque des revendications précédentes, caractérisé en ce que ladite réaction est mise en oeuvre dans un solvant sélectionné dans le groupe consistant en eau, alcool aliphatique et leurs mélanges dans un rapport pondéral amine/solvant compris entre 1/100 et 30/100, la température de réaction étant comprise entre 25 et 150°C.

5. Procédé selon l'une quelconque des revendications précédentes, caractérisé en ce que la quantité de catalyseur est comprise entre 0,1 et 50 parties en poids pour 100 parties d'amine.

6. Procédé selon l'une quelconque des revendications précédentes, caractérisé en ce que le rapport molaire H₂O₂/amine est compris entre 0,9 et 1,2.
